Europäisches Patentamt

European Patent Office

Office européen des brevets

(19)

(11) Veröffentlichungsnummer: **0 355 444 B1**

(12) # EUROPÄISCHE PATENTSCHRIFT

(45) Veröffentlichungstag der Patentschrift: **16.12.92**

(21) Anmeldenummer: **89113730.9**

(22) Anmeldetag: **25.07.89**

(51) Int. Cl.⁵: **A41D 13/00**, A61B 19/00

(54) **Gesichtsmaske.**

(30) Priorität: **27.07.88 DE 8809551 U**
**17.03.89 DE 8903326 U**

(43) Veröffentlichungstag der Anmeldung:
**28.02.90 Patentblatt 90/09**

(45) Bekanntmachung des Hinweises auf die
Patenterteilung:
**16.12.92 Patentblatt 92/51**

(84) Benannte Vertragsstaaten:
**AT BE CH DE ES FR GB GR IT LI LU NL SE**

(56) Entgegenhaltungen:
**EP-A- 0 242 988**
**DE-U- 8 523 200**
**US-A- 2 056 753**
**US-A- 3 834 384**
**US-A- 3 885 558**

(73) Patentinhaber: **Dunsch-Herzberg, Renate**
**Holmer Strasse 165**
**W-2000 Wedel/Holstein(DE)**

Patentinhaber: **Voss, Gudrun**
**Cranz 26**
**W-2081 Hetlingen(DE)**

(72) Erfinder: **Herzberg, Wolfgang, Dr.**
**Holmer Strasse 165**
**W-2000 Wedel/Holstein(DE)**

(74) Vertreter: **Richter, Werdermann & Gerbaulet**
**Neuer Wall 10**
**W-2000 Hamburg 36(DE)**

## Beschreibung

Die Erfindung betrifft eine Gesichtsmaske zum Schutz des Operateurs, des medizinischen Personals u.dgl. gegen Sekret- und Bluteinwirkung von an der Immunschwächekrankheit Aids erkrankten Patienten, bestehend aus einem Maskenzuschnitt mit seitlich angebrachten Befestigungsbändern und mit einem an dem oberen Rand des Maskenzuschnittes ausgebildeten Dichtungsrand aus einer gesichtsseitig ausgebildeten, selbstklebenden Fläche, die im Nichtgebrauchszustand mittels eines abziehbaren Schutzblattes abgedeckt ist.

Eine solche Gesichtsmaske ist aus der US-A-3 834 384 bekannt. Diese Gesichtsmaske besteht aus einem Maskenzuschnitt mit seitlich angebrachten Befestigungsbändern und weist einen oberen Randabschnitt auf, der als Dichtungsrand mit einer gesichtsseitig vorgesehenen,selbstklebenden Besichtung versehen ist, wobei diese sich über die gesamte Fläche des Randabschnittes erstreckt. Ein derart ausgebildeter Dichtungsrand mit seiner sich über die gesamte Randfläche erstreckenden,selbstklebenden Beschichtung paßt sich bei angelegter Gesichtsmaske nicht dem Gesichtsmuskelspiel des Operateurs an, weil der Dichtungsrand vollflächig auf die Gesichtshaut aufgeklebt ist und keine Möglichkeit besitzt, sich einem Muskelspiel durch Dehnung anzupassen. Gesichtsmasken mit einem derartigen Dichtungsrand werden im Laufe einer z.B. mehrstündigen Operation undicht, wobei diese Undichtigkeit darauf zurückzuführen ist, daß durch das Gesichtsmuskelspiel des Operateurs sich der Dichttungsstreifen abschnittsweise abhebt, was durch Schweißbildung noch gefördert wird. Hinzu kommt, daß, wenn die Gesichtsmaske zu straff auf die Gesichtshaut aufgeklebt ist, dies vom Operateur als lästig empfunden wird, so daß die Gesichtsmuskeln dem entgegenarbeiten, um einen auftretenden Zug und Druck auszuschalten, was ebenfalls zu einer Lockerung des Dichtungsrandes und damit zu Undichtigkeiten führt, so daß auf der Stirn des Operateurs gebildeter Schweiß durch die undichten Stellen im oberen Randbereich der Gesichtsmaske in den Wangen- und Mundbereich dringen kann, was gerade bei längeren Operationen und wo es keine Möglichkeit zur Unterbrechung gibt als störend empfunden Wird, auch wenn während der Operation von der Operationsschwester sich auf der Stirn des Operateurs bildender Schweiß abgetupft wird. Außerdem kann Atemluft durch die undichten Stellen im oberen Randbereich der Gesichtsmaske austreten. Da aber eine Gesichtsmaske für einen Operateur so auf die Gesichtshaut aufgesetzt sein soll, daß von außen durch Schweißbildung keine Feuchtigkeit in den Wangen- und Mundbereich dringen und keine Atemluft nach außen strömen kann, ist es erforderlich, daß der Dichtungsstreifen fest und straff sitzend auf die Gesichtshaut aufgeklebt wird, was zu den voranstehend geschilderten störenden Eigenschaften führt.

Aufgrund der sich ausbreitenden Immunschwächekrankheit Aids reichen bisher übliche Maßnahmen zum Schutz des medizinischen Personals nicht mehr aus. So ist es u.a. erforderlich, daß die Augen des medizinischen Personals vor Kontakt mit Sekreten und Blut von Patienten geschützt werden. Die hierzu eingesetzten Brillen erfüllen zwar ihre Schutzfunktion, schaffen jedoch eine Vielzahl von neuen Problemen:

- Brillen sind personengebundene Gegenstände, was bedeutet, daß der jeweilige Besitzer einen weiteren Gegenstand pflegen und betreuen muß. Der Kleidungswechsel im operativen Bereich ist als Ursache für den Verlust persönlicher Gegenstände hinlänglich bekannt.

  Brillen sind auch bei sorgfältiger Pflege keineswegs keimarme Gegenstände, was bedeutet, daß bei einer Operation die Brille des Chirurgen zu einer Infektionsursache werden kann.
- Ein Nichtbrillenträger wird sich nur mühsam in die Benutzung einer Brille hineinfinden; diese jedoch unter Umständen aufgrund des Ungewohnten häufig nicht aufsetzen.
- Die Kombination aus Mundschutz und Brille führt zum Beschlagen der Brillengläser, ein Problem, das auch für den mikroskopierenden Chirurgen bis heute ungelöst ist.

Gesichtsmasken mit einer Sichtfläche zum Schützen der Augen und der Stirn sind bekannt. So beschreibt die US-A-2 056 753 eine Gesichtsmaske, die einen Folienzuschnitt aus einem glasklaren Werkstoff als Sichtfläche, zwei Befestigungsbänder, zwischen denen die Sichtfläche angeordnet ist, und einen Mund- und Kinnschutz aus einem Baumwollgewebe aufweist. Zu beiden Seiten der Sichtfläche sind Gewebeabschnitte zur Abdeckung der Ohren vorgesehen. Von den beiden Befestigungsbändern ist ein Befestigungsband durch den Rand des den Mundschutz bildenden Abschnittes so geführt, daß nach dem Anlegen der Gesichtsmaske und nach einem Anziehen des Befestigungsbandes an seinen beiden freien Enden sich der untere Gewebeabschnitt der Gesichtsmaske um Mund und Kinn schützend anlegt. Eine besondere abdichtende Randausgestaltung ist bei dieser Gesichtsmaske nicht vorgesehen.

Durch die DE-U-88 04 403 ist eine Atemschutzmaske, bestehend aus einem Vliesträger, einem Filter und einem Abdeckvlies bekannt, bei der zumindest Teilbereiche oder ein Teilbereich des Abdeckvlieses aus in der Luft, vorzugsweise in der ausgeatmeten Atemluft enthaltene Feuchtigkeit, absorbierendes Vliesmaterial bestehen. Mit einer derart ausgebildeten Gesichtsmaske soll Feuchtigkeit in der ausgeatmeten Luft zur Verhinderung des Beschlagens von Brillengläsern des Atemschutzmasken-Trägers gebunden werden.

Die DE-A-29 38 720 beschreibt eine Atemhalbmaske für den Einmalgebrauch aus einem gefalteten Kunststoffaservlies als Filtermaterial in einer Form, die einen Sitz über der Nase, den Wangen und dem Kinn des Benutzers sichert und einem einwärtsgerichteten, als Dichtlippe ausgebildeten Dichtstreifen aus dem gleichen Material sowie Kopfbändern, die am Rand befestigt sind. Zwischen dem mit fixierten Längsfalten und in eine dem Gesicht angepaßte Hohlform angebrachten Maskenkörper und der umlaufend ausgebildeten Dichtlippe, die mit einem der Nase gegenüber angeordneten Schlitz versehen ist, ist ein mit einem Nasenausschnitt versehener Verstärkungsrahmen aus einem hartelastischen Kunststoff mit seitlich nach außen überstehenden Laschen zur Befestigung der Kopfbänder eingelegt, wobei der Maskenkörper, der Verstärkungsrahmen und die Dichtlippe,einen Rand bildend, dicht miteinander verbunden sind. Eine derartige Atemhalbmaske ist für den Einmalgebrauch vorgesehen und es soll mit ihr eine gute Abdichtung und eine gesicherte Filterleistung garantiert werden, wobei die Maskenformgebung und deren Formstabilität bei Beanspruchung und bei Durchfeuchtung erhalten bleiben soll. Diese Atemhalbmaske weist aufgrund ihrer konstruktiven Ausgestaltung eine hohe Formstabilität auf, die dadurch erhalten wird, daß in den Rand zwischen den beiden Teilen Maskenkörper und Dichtlippe ein Verstärkungsrahmen aus einem hartelastischen Kunststoff eingelegt und mit verschweißt bzw. verklebt ist. Der Maskenzuschnitt selbst weist jedoch innenseitig und an seinem oberen Rand keine Dichtlippe aus einem Schaumstoffstreifen mit einer Klebefläche auf, denn die Abdichtung soll nicht über eine Dichtlippe oder einen Dichtstreifen erfolgen, sondern durch Maskenformgebung und Formstabilität

Nach der DE-A-36 01 449 ist eine chirurgische Gesichtsmaske bekannt, die einen Barrierestreifen auf dem Tuch, welches die Gesichtsmaske bildet, aufweist, der das Durchdringen von Feuchtigkeit durch das Tuch verhindern soll, wobei der Barrierestreifen ein Kunstharz-Material und einen Polyester-Schaumstoff aufweist. Mit dieser Gesichtsmaske soll eine nicht kontaminierende chirurgische Gesichtsmaske erhalten werden, die in ihrer Eignung zur Verhinderung des Austritts von Feuchtigkeitsdampf durch den Oberteil der Maske überlegen sein soll und bei der außerdem ein freier Luftdurchtritt zur Erleichterung des Atems möglich sein soll. Diese chirurgische Gesichtsmaske weist ein gefaltetes Gesichtstuch auf, welches den Mund und die Nase des Trägers abdeckt und das aus einem Kunststoff hergestellt ist, der für teilchenartigen Stoff undurchdringlich ist. An der oberen Innenseite des gefalteten Gesichtstuches befindet sich eine laminierte Feuchtigkeitsbarriere, die durch Ultraschallschweißung damit heißversiegelt ist. Eine Schicht dieser Feuchtigkeitsbarriere weist einen Polyesterschaum auf, der durch eine Flamme auf eine weitere Innenschicht eines 100%ig ungewebten Polyester-Materials aufgebracht ist. Nach der Heißlaminierung läuft die Schichtzusammensetzung durch Kalandrierwalzen hindurch und wird permanent zusammengedrückt. Die Porosität der geschichteten Zusammensetzung soll somit verringert und der Durchtritt von Dampf entsprechend beschränkt werden. Der bei dieser Gesichtsmaske vorgesehene Barrierestreifen besteht ausschließlich aus einem Kunstharz-Material und einem Polyester-Schaumstoff. Einen Schaumstoffstreifen mit einer Beschichtung aus einem Selbstklebemittel als Barrierestreifen einzusetzen ist bei dieser Bekannten Gesichtsmaske nicht vorgesehen.

Ein weiterer chirurgischer Kopfschutz und Gesichtsschutz ist durch die DE-A-32 01 291 bekannt. Dieser Kopf- und Gesichtsschutz besteht aus einer Kopfschutzhaube bzw. Kopfschutzkappe und aus einer Gesichtsschutzmaske, die jeweils mit einem angeklebten oder auf sonst eine andere Weise lösbar verbundenen Streifen aus biegsamem Material versehen sein können, der abgezogen und zum Halten einer Brille in einer vorgegebenen Position an anderer Stelle wieder angebracht werden kann. Darüber hinaus weist die zum Kopfschutz- und Gesichtsschutzsystem gehörende Kopfschutzhaube oder Kopfschutzkappe auch noch ein feuchtigkeitsabsorbierendes Element auf, das im Kleberverband lösbar auf die Außenfläche der Kopfschutzhaube bzw. Kopfschutzkappe aufgeklebt ist, das abgezogen werden kann und das schließlich an anderer Stelle im Inneren der Kopfschutzhaube oder Kopfschutzkappe wieder angebracht und befestigt werden kann.

Bei diesem Kopf- und Gesichtsschutz handelt es sich um ein zweiteilig ausgebildetes System, das aus einer Kappe bzw. Haube und einer Mundschutzmaske für Brillenträger besteht. Außerdem ist dieser Kopfschutz mit einer Brillenhaltevorrichtung versehen, die lösbar an einem der Kopf- und Gesichtsschutzteile befestigt ist. Im wesentlichen geht es bei diesem Kopfschutz um eine Brillenhaltevorrichtung bei einer chirurgischen Gesichtsschutzmaske, wobei die Gesamtausgestaltung derart ist, daß die Brille an der vorgesehenen Stelle auf der Nase des Brillenträgers gehalten wird. Das bei diesem Kopf- und Gesichtsschutz vorgesehene feuchtigkeitsabsorbierende Element, das im Kleberverband lösbar auf die Außenfläche der Kopfschutzhaube bzw. Kopfschutzkappe aufgeklebt ist, das abgezogen und an anderer Stelle im Inneren der Kopfschutzhaube oder Kopfschutzkappe wieder angebracht und befestigt werden kann, besteht aus einem Polster aus einem atemabsorbierenden Material, das an der Außenfläche des Kopfwölbungsbereiches der Kappe bzw. Haube befestigt und leicht abgezogen werden kann, um eine Befestigung am Rand der Haube vornehmen zu können. Dieses atemabsorbierende Polster kann auch an der Innenseite des Randes der Haube befestigt werden, um als atemabsorbierender Barrierestreifen zu dienen. Die Anbringung eines Schaumstoffstreifens mit einer außenseitigen Beschichtung mit einem Selbstklebemittel , um nach dem Anlegen der Gesichtsmaske deren oberen Rand auf der Gesichtshaut festkleben zu können, ist bei diesem Kopf- und Gesichtsschutz für Chirurgen nicht vorgesehen.

Eine weitere Gesichtsmaske für den einmaligen Gebrauch ist durch die EP-A-0051616 bekannt. Diese Gesichtsmaske ist mit einem Hauptteil versehen, der die Nase und den Mund bedecken soll; ferner sind Einrichtungen zum Befestigen dieses Hauptteils über der Nase und dem Mund vorgesehen. Der Hauptteil der Gesichtsmaske ist dabei als Filtermedium ausgebildet und besteht aus einer Bahn mit einem Gehalt an fibrillierten Electret-Fasern, wobei diese Bahn ein Gewicht von 6 bis 50 g/m$^2$ aufweist, wobei die genannten Fasern eine durchschnittliche Weite von mindestens 10 Mikrometer aufweisen. Bei dieser Gesichtsmaske handelt es sich jedoch nicht um eine chirurgische Gesichtsmaske mit einem Barrierestreifen, der das Durchdringen von Feuchtigkeit verhindern oder behindern soll, sondern ausschließlich geht es um die Schaffung eines speziell ausgebildeten textilen Flächengebildes, aus dem die Gesichtsmaske hergestellt wird.

Der Erfindung liegt die Aufgabe zugrunde, eine Gesichtsmaske der eingangs genannten Art zum Schutz des Operateurs und des medizinischen Personals gegen Sekret- und Bluteinwirkung von an der Immunschwächekrankheit Aids erkrankten Patienten zu schaffen, mit der nicht nur ein wirksamer Schutz erreicht wird, sondern bei der die feuchte Atemluft nicht am Oberrand der Maske entweichen kann und die des weiteren als Alternative mit einem Sichtschutz als Einmal-Artikel kombinierbar ist.

Diese Aufgabe wird durch die im Anspruch 1 gekennzeichneten Merkmale gelöst.

Mit einer derartigen Ausgestaltung ist eine Gesichtsmaske geschaffen, bei der die feuchte Atemluft nicht am Oberrand der Maske entweichen kann. Dies wird verhindert durch den am oberen Rand des Maskenzuschnittes der Gesichtsmaske angeordneten Schaumstoffstreifen, der gesichtsseitig zugewandt mit einer Klebeschicht versehen ist, so daß aufgrund der leichten Verformbarkeit des Maskenzuschnittes und seines Schaummstoffstreifens der Maskenzuschnitt mit seinem oberen, innenseitig die Klebeschicht tragenden Rand über die Nasen- und auf die Wangenpartie unterhalb der Augen geklebt wird, so daß ein Mund- und Nasenschutz gewährleistet ist. Dadurch, daß der Schaumstoffstreifen aus einem Faltstreifen besteht, ist eine Anformung an Nase- und Wangenpartie möglich. Es wird somit ein dichter Abschluß am Oberrand der Gesichtsmaske zur Nasen- und Wangenpartie erreicht. Des weiteren ist die Gesichtsmaske mit einem Sichtschutz als Einmal-Artikel kombinierbar.

Der am inneren Oberrand der Gesichtsmaske angeordnete Schaumstoffstreifen, der auch als Gummi-/Kunststoff-Falz ausgebildet sein kann und der mit einem hautfreundlichen Klebestreifen bzw. mit einer Beschichtung eines Klebemittels versehen ist, ermöglicht die luftdichte Abdichtung des Oberrandes der Gesichtsmaske gegen die Gesichtshaut.

Nach einer weiteren Ausführungsform der Erfindung ist außen am Oberrand der Gesichtsmaske eine glasklare, flexible und möglichst reflexarme Kunststoff-Folie aufgenäht bzw. aufgeklebt. Diese bedeckt in ihren Ausmaßen großzügig die Augen und Teile der Stirn. Dabei sollte die Kunststoff-Folie fest an der Stirn anliegen, um von oben einfallendes Licht möglichst wenig als störende Reflexe wirksam werden zu lassen. Dabei ist es vorteilhaft, den oberen Rand der Kunststoff-Folie in Richtung zur Gesichtshaut leicht abzuwinkeln .

Des weiteren sieht die Erfindung eine Gesichtsmaske mit einer Kopfhaube vor, die als sogenannte Astronautenhaube ausgebildet und die mit der Gesichtsmaske lösbar oder fest verbunden ist, wobei die Kopfhaube außenseitig einen an ihrem oberen Rand fest oder lösbar befestigten schirmartigen Zuschnitt als Sicht- und Reflexionsschutz aufweist, wobei der Zuschnitt aus einer schwarz oder anderweitig dunkel eingefärbten , opaken oder mattierten Kunststoff-Folie besteht. Dieser Schirm als Schutz gegen störende Reflexe ist entweder auf die Einmal-Haube,z.B. vom Typ Astronaut, aufgenäht bzw. aufgeklebt oder wird separat mit einem Band um die Stirn gelegt.

Vorteilhafte Ausgestaltungen der Erfindung sind in den Unteransprüchen gekennzeichnet.

Ausführungsbeispiele der Erfindung werden nachstehend anhand der Zeichnung näher erläutert. Es zeigen

Fig. 1 in einer schaubildlichen Ansicht eine Gesichtsmaske mit an ihrem Maskenzuschnitt angebrachtem Schaumstoffstreifen,

Fig. 2 in einer schaubildlichen Ansicht die Gesichtsmaske mit am oberen Rand ihres Maskenzuschnittes angebrachter Sichtfläche,

Fig. 3 einen Abschnitt des Maskenzuschnittes mit dem Schaumstoffstreifen in einer schaubildlichen Ansicht,

Fig. 4 einen Abschnitt des Maskenzuschnittes mit einem Faltstreifen in einer schaubildlichen Ansicht und

Fig. 5 eine Einmal-Haube in einer schaubildlichen Ansicht.

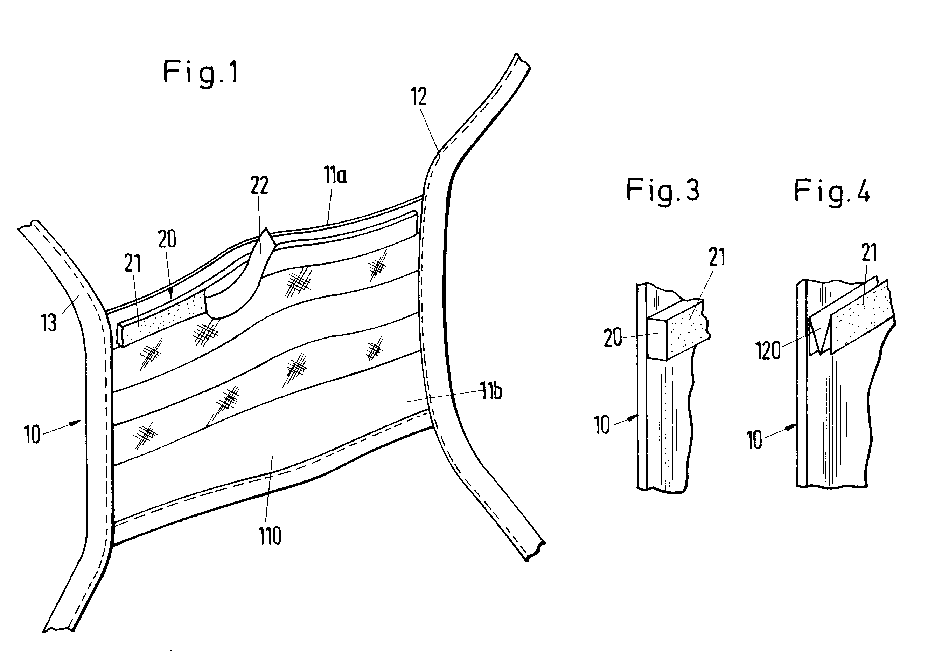

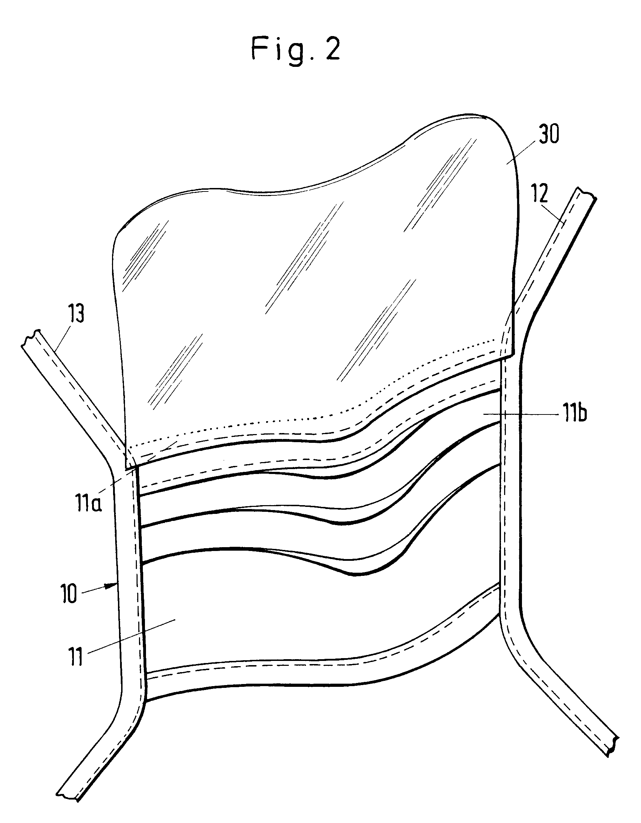

Die in Fig. 1 dargestellte und mit 10 bezeichnete Gesichtsmaske ist in an sich bekannter Weise ausgebildet und besteht aus einem Maskenzuschnitt 110, der an seinen gegenüberliegenden Randbereichen je ein Befestigungsband 12,13 trägt Der Maskenzuschnitt 110 selbst besteht aus einem Flächengebilde aus einen geeigneten Gewebe od.dgl.. Der obere Rand des Maskenzuschnittes 110 ist mit 11a und die Innenseite des Maskenzuschnittes 110 mit 11b bezeichnet.

Benachbart zu seinem oberen Rand 11a weist der Maskenzuschnitt 110 , und zwar an seiner Innenseite 11b, einen Schaumstoffstreifen 20 auf, der mit einer bei 21 angedeuteten Klebefläche versehen ist, die aus einer Beschichtung mit einem Selbstklebemittel besteht. Diese Klebeschicht 21 ist mittels eines abziehbaren Schutzblattes 22 abgedeckt. Als Werkstoff für den Streifen 20 können alle geeigneten Schaumkunststoffe zur Anwendung gelangen. Wird die Gesichtsmaske 10 angelegt, dann wird vorher das Schutzblatt 22 vom Schaumstoffstreifen 20 abgezogen und der obere Rand 11a des Maskenzuschnittes 110 mit seinem Schaumstoffstreifen 20 an die Gesichtshaut angedrückt, wobei die Klebeschicht auf der Gesichtshaut oberhalb der Nase und auf den Wangenpartien unterhalb der Augen angedrückt wird, wodurch ein luftdichter Abschluß zur Gesichtshaut erreicht wird.

Neben der Verwendung eines glatten Schaumstoffstreifens 20 entsprechend Fig. 3 besteht auch die Möglichkeit, einen Falt- bzw. Falzstreifen 120 gemäß Fig. 4 zu verwenden, der ebenfalls aus Schaumkunststoffen oder anderen geeigneten federnd-elastischen , insbesondere weichen Kunststoffen besteht.

Der Maskenzuschnitt 110 der Gesichtsmaske 10 weist außenseitig und an seinem oberen Rand 11a als Sichtfläche einen Folienzuschnitt 30 auf, der aus einem glasklaren, flexiblen und reflexionsarmen Kunststoff besteht. Dieser Folienzuschnitt 30 ist mittels einer Klebe- oder Nähverbindung mit dem Maskenzuschnitt 110 der Gesichtsmaske 10 verbunden und bedeckt in seinen Ausmaßen großzügig die Augen und Teile der Stirn. Der Folienzuschnitt soll dabei fest an der Stirn anliegen, um von oben einfallendes Licht möglichst wenig als störende Reflexe wirksam werden zu lassen (Fig.2).

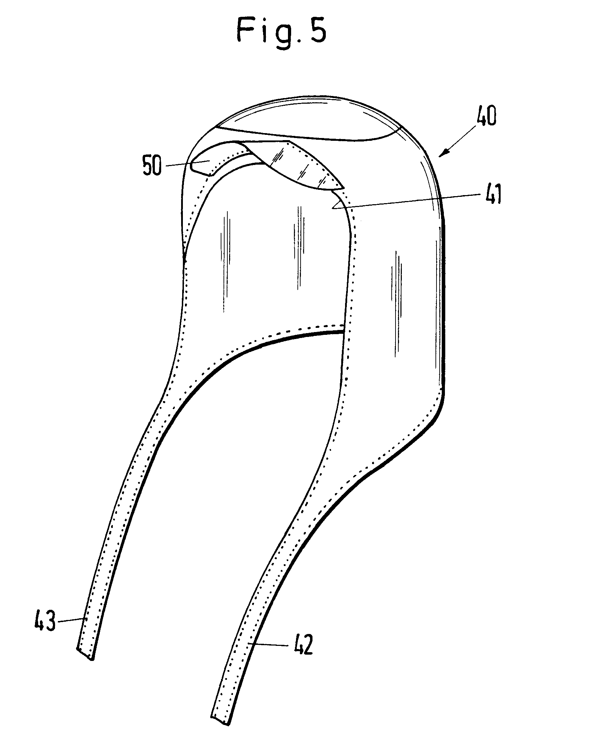

Die Gesichtsmaske 10 kann auch in Verbindung mit einer Kopfthaube 40 eingesetzt werden. Eine derartige Kopfhaube oder Einmal-Haube vom Typ Astronaut ist in Fig.5 dargestellt. Die Gesichtsmaske 10 ist dabei mit der Kopfhaube 40 lösbar oder fest verbunden. Die Befestigungsbänder der Kopfhaube 40 sind mit 42,43 bezeichnet. Ist die Gesichtsmaske 10 mit der Kopfhaube 40 fest verbunden, dann wird die Kopfhaube 40 durch überziehen über den Kopf angelegt. Ist dagegen die Gesichtsmaske 10 mit der Kopfhaube 40 lösbar verbunden, dann erfolgt nach dem Aufsetzen und der Befestigung der Kopfhaube 40 die Anbringung der Gesichtsmaske 10. Die Kopfhaube 40 weist außenseitig an ihrem oberen Rand 41 einen schirmartigen Zuschnitt 50 als Sicht- und Reflexionsschutz auf, wobei dieser schirmartige Zuschnitt 50 mit dem oberen Rand 41 der Kopfhaube 40 fest oder lösbar befestigt sein kann. Der schirmartige Zuschnitt 50 besteht aus einer schwarzen oder anderweitig dunkelfarbenen , opaken oder mattierten Kunststofffolie. Die Befestigung des schirmartigen Zuschnittes 50 an dem oberen Rand 41 der Kopfhaube 40 erfolgt mittels Kleb- oder Nähverbindungen, wenn eine feste Verbindung gewünscht wird. Soll dagegen der schirmartige Zuschnitt 50 lösbar mit der Kopfhaube 40 verbunden sein, dann ist der schirmartige Zuschnitt 50 an dem oberen Rand 41 der Kopfhaube 40 mittels eines in an sich bekannter Weise ausgebildeten Klettenverschlusses befestigt. Dieser,nach der Haftfähigkeit, dem typischen Merkmal der Klette, benannte Verschluß besteht aus zwei Teilen, die nach einem Spezialverfahren hergestellt und ausgerüstet werden und von denen das eine Teil an dem schirmartigen Zuschnitt 50 und das andere Teil an dem oberen Rand 41 der Kopfhaube 40 befestigt ist. Das eine Teil besteht aus einem Belag aus kleinen Widerhaken, während das andere Teil mit einer flauschigen Faserschicht ausgerüstet ist, so daß die Häkchen in dieser flauschigen Faserschicht fest haften bleiben, wenn beide Teile ,also hier schirmartiger Zuschnitt und oberer Rand der Kopfhaube, miteinander in Verbindung gebracht werden. Wird auf den angesetzten schirmartigen Zuschnitt 50 ein leichter Druck gegen den oberen Rand 41 der Kopfhaube 40 ausgeübt, wobei der obere Rand 41 der Kopfhaube 40 mit der flauschigen Faserschicht ausgerüstet ist, während der schirmartige Zuschnitt den Belag aus kleinen Widerhaken trägt, greifen die Häkchen in die Faserschicht ein und halten beide Teile , nämlich schirmartigen Zuschnitt und Kopfhaube, zusammen. Dieser Klettenverschluß ist leicht zu öffnen, indem der schirmartige Zuschnitt 50 mit leichtem Ruck dann von dem oberen Rand 41 der Kopfhaube 40 abgezogen wird. Auch der schirmartige Zuschnitt 50 kann an seinen seitlichen Endbereichen mit Befestigungsbändern versehen sein, wodurch die Möglichkeit gegeben ist, nach Anlegen der Kopfhaube zusätzlich den schirmartigen Zuschnitt anzubringen.

Die Kunststoff-Folie, aus der der Zuschnitt 50 besteht, weist eine ausreichende Eigensteifigkeit auf, um dem Zuschnitt 50 die schirmartige Form zu geben.

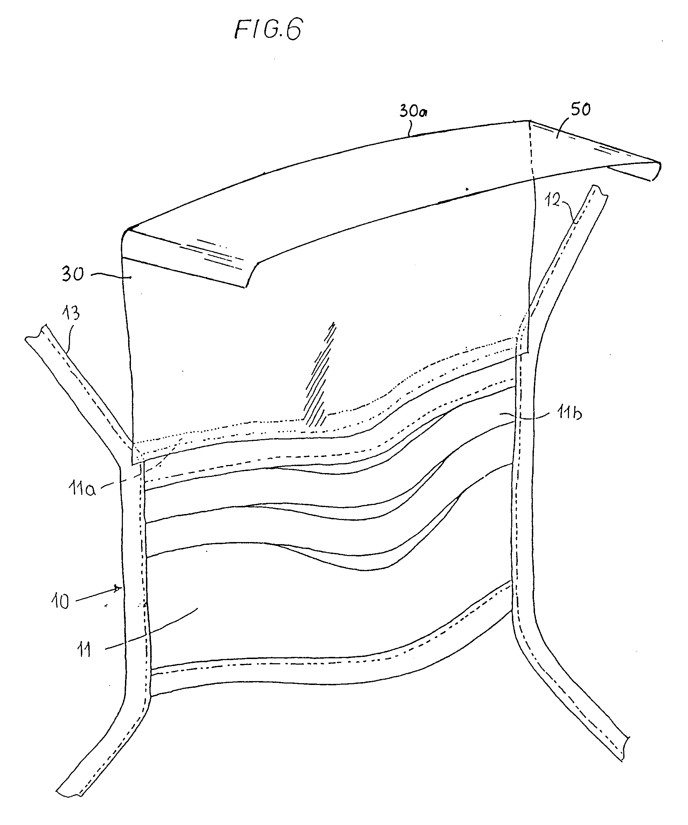

Nach einer weiteren Ausführungsform ist der als Sichtschutz dienende Folienzuschnitt 30 an dem Maskenzuschnitt 110 der Gesichtsmaske 10 mit einem schirmartigen Zuschnitt 50 als Blendschutz versehen, wobei dieser schirmartige Zuschnitt 50 an dem oberen Rand 30a des Folienzuschnittes 30 vermittels Kleb- oder Schweißverbindungen oder anderer geeigneter Verbindungen angebracht ist (Fig.6). Auch eine einstückige Ausgestaltung des schirmartigen Zuschnittes 50 und des Folienzuschnittes 30 ist möglich. In diesem Falle besteht der gesamte Formkörper aus einem glasklaren Werkstoff, insbesondere Kunststoff, wobei der den Blendschutz bildende Abschnitt des den Sichtschutz und den Blendschutz bildenden Formkörpers dann eingefärbt ist, um eine Blendschutzwirkung zu erreichen. Als Werkstoff für diesen Formkörper findet der zur Herstellung des Maskenzuschnittes 110 verwendete Werkstoff Anwendung.

Der schirmartige Zuschnitt 50 an dem Maskenzuschnitt 110 oder an der Kopfhaube 40 kann sowohl an dem Folienzuschnitt 30 als auch an der Kopfhaube 40 lösbar befestigt sein. Diese lösbare Befestigung hat den Vorteil, den Zuschnitt 50 abnehmen zu können, wenn kein Blendschutz benötigt wird oder ein Blendschutz hinderlich sein könnte. Außerdem besteht die Möglichkeit, durch die lösbare Anbringung Zuschnitte unterschiedlicher Größe einsetzen und verwenden zu können, wenn dies erforderlich werden sollte. Als lösbare Verbindung kann z.B. ein Klettenverschluß eingesetzt werden; jedoch auch andersartig ausgebildete lösbare Verbindungsmittel, wie z.B. Haftklebemittel, Klemmverbindungen od.dgl. können zur Anwendung gelangen.

**Patentansprüche**

1. Gesichtsmaske zum Schutz des Operateurs, des medizinischen Personals u.dgl. gegen Sekret- und Blut-Einwirkung von an der Immunschwächekrankheit Aids erkrankten Patienten, bestehend aus einem Maskenzuschnitt (110) mit seitlich angebrachten Befestigungsbändern (12,13) und mit einem an dem oberen Rand (11a) des Maskenzuschnittes ausgebildeten Dichtungsrand aus einer gesichtsseitig ausgebildeten, selbstklebenden Fläche, die im Nichtgebrauchszustand mittels eines abziehbaren Schutzblattes (22) abgedeckt ist, dadurch gekennzeichnet, daß der Dichtungsrand des Maskenzuschnittes (110) der Gesichtsmaske (10) aus einem innenseitig (11b) an dem Maskenzuschnitt angeordneten, quer zur Längsrichtung gefalteten Schaumstoffstreifen (120) mit einer gesichtsseitig zugekehrten Klebefläche (21) aus einer Beschichtung mit einem Selbstklebemittel besteht.

2. Gesichtsmaske nach Anspruch 1, dadurch gekennzeichnet, daß der Dichtungsrand des Maskenzuschnittes (110) der Gesichtsmaske (10) aus einem gesichtsseitig am Maskenzuschnitt angeordneten, quer zur Längsrichtung gefalteten Schaumstoffstreifen (120) mit einer gesichtsseitig zugekehrten Klebefläche (21) aus einer Beschichtung mit einem Selbstklebemittel besteht, wobei der Maskenzuschnitt (110) außenseitig und an seinem oberen Rand (11a) einen Folienzuschnitt (30) als Sichtfläche aus einem glasklaren, flexiblen und reflexionsarmen Kunststoff aufweist, der mittels einer Kleboder Nähverbindung mit dem Maskenzuschnitt (110) verbunden ist.

3. Gesichtsmaske nach Anspruch 1, dadurch gekennzeichnet, daß der Dichtungsrand des Maskenzuschnittes (110) der Gesichtsmaske (10) aus einem gesichtsseitig am Maskenzuschnitt angeordneten, quer zur Längsrichtung gefalteten Schaumstoffstreifen (120) mit einer gesichtsseitig zugekehrten Klebefläche (21) aus einer Beschichtung mit einem Selbstklebemittel besteht, und daß die Gesichtsmaske (10) mit einer Kopfhaube (40) lösbar oder fest verbunden ist, wobei die Kopfhaube (40) außenseitig einen an ihrem oberen Rand (41) fest befestigten, schirmartigen Zuschnitt (50) als Sicht- und Reflexionsschutz aufweist, wobei der Zuschnitt (50) aus einer schwarz oder anderweitig dunkel eingefärbten, opaken oder mattierten Kunststoffolie besteht und an dem oberen Rand (41) der Kopfhaube (40) mittels Kleb- oder Nähverbindungen befestigt ist.

4. Gesichtsmaske nach Anspruch 1, dadurch gekennzeichnet, daß der Dichtungsrand des Maskenzuschnittes (110) der Gesichtsmaske (10) aus einem gesichtsseitig am Maskenzuschnitt angeordneten, quer zur Längsrichtung gefalteten Schaumstoffstreifen (120) mit einer gesichtsseitig zugekehrten Klebefläche (21) aus einer Beschichtung mit einem Selbstklebemittel besteht, und daß die Gesichtsmaske (10) mit einer Kopfhaube (40) lösbar oder fest verbunden ist, wobei die Kopfhaube (40) außenseitig einen an ihrem oberen Rand (41) lösbar befestigten, schirmartigen Zuschnitt (50) als Sicht- und Reflexionsschutz aufweist, wobei der Zuschnitt (50) aus einer schwarz oder anderweitig dunkel eingefärbten, opaken oder mattierten Kunststoffolie besteht und an dem oberen Rand (41) der Kopfhaube (40) mittels eines Klettenverschlusses befestigt ist.

5. Gesichtsmaske nach einem der Ansprüche 2 bis 4, dadurch gekennzeichnet, daß der schirmartige Zuschnitt (50) an seinen seitlichen Endbereichen Befestigungsbänder aufweist.

## Claims

1. Face mask for protecting the operating surgeon, the medical staff and others against the effects of secretions and blood of patients who have contracted the immunodeficiency disease AIDS, comprising a mask blank (110) with laterally fitted attachment tapes (12,13) and with a sealing rim formed on the upper border (11a) of the mask blank of a self-adhesive surface constructed on the side of the face which, in the non-used state, is covered by means of a peelable protective sheet (22), characterized in that the sealing rim of the mask blank (110) of the face mask (10) is comprised of a cellular plastic strip (120) disposed inside (11b) the mask blank and folded transversely to the longitudinal direction and having an adhesive surface (21) turned towards the face side of a coating with a pressure-sensitive adhesive.

2. Face mask according to Claim 1, characterized in that the sealing rim of the mask blank (110) of the face mask (10) comprises a strip of cellular plastic disposed on the face side on the mask blank and folded transversely to the longitudinal direction and having an adhesive surface (21) comprised of a coating with a pressure-sensitive adhesive, in which the mask blank (110), on the outside and on its upper border (11a), is provided with a foil blank (30) in the form of a viewing area of a pellucid, flexible plastic of low reflectivity, which, by means of an adhesive or sewn connection, is connected to the face mask blank (110).

3. Face mask according to Claim 1, characterized in that the sealing rim of the mask blank (110) of the face mask (10) comprises a cellular plastic strip (120) disposed on the face side on the mask blank and folded transversely to the longitudinal direction having an adhesive surface (21) turned towards the face side of a coating with a pressure-sensitive adhesive, and in that the face mask (10) is detachably or rigidly connected to a hood for the head (40), the hood (40) being provided on the outside with a vizor-like blank (50) rigidly connected to its upper rim (41) as a visual and reflection protection, while the blank (50) is comprises of a black or otherwise dark-coloured opaque or dully polished plastic sheeting and is attached to the upper rim (41) of the hood (40) with the aid of adhesive or sewn connections.

4. Face mask according to Claim 1, characterized in that the sealing rim of the mask blank (110) of the face mask (10) comprises a strip of cellular plastic (120) disposed on the face side of the mask blank and folded transversely to the longitudinal direction and having an adhesive surface (21) turned towards the face of a coating with a pressure-sensitive adhesive, and in that the face mask (10) is detachably or rigidly connected to a hood (40), wherein the hood (40) is provided on the outside with a vizor-like blank (50) detachably connected to its upper rim (41) as a visual and reflection protection, while the blank (50) is comprised of a black or otherwise dark-coloured, opaque or dully polished plastic sheeting and is attached to the upper rim (41) of the hood by means of a Velcro closure.

5. Face mask according to any of Claims 2 to 4, characterized in that the vizor-like blank (50) is provided with attachment tapes on its lateral terminal areas.

## Revendications

1. Masque pour visage pour la protection de l'opérateur, du personnel médical ou équivalent contre les effets des sécrétions et du sang de patients souffrant du syndrome immunodéficitaire SIDA, composé d'une pièce découpée pour masque (110) avec des bandes de fixation (12, 13) fixées sur les côtés et avec un bord d'étanchéité, formé sur le bord supérieur (11a) de la pièce découpée pour masque, composé d'une surface autocollante formée côté visage qui est recouverte, à l'état de non utilisation, par une feuille de protection pelliculable (22), **caractérisé en ce** que le bord d'étanchéité de la pièce découpée pour masque (110) du masque pour visage (10) est constituée par une bande de mousse (120), placée sur le côté intérieur (11b) de la pièce découpée pour masque en étant pliée dans le sens transversal par rapport au sens de la longueur, avec une surface collante (21), tournée vers le visage, constituée par une enduction avec une substance autocollante.

2. Masque pour visage selon la revendication 1, **caractérisé en ce** que le bord d'étanchéité de la pièce découpée pour masque (110) du masque pour visage (10) est constituée par une bande de mousse (120), placée du côté visage sur la pièce découpée pour masque en étant pliée dans le sens transversal par rapport au sens de la longueur, avec une surface collante (21), tournée vers le visage, constituée par une enduction avec une substance autocollante, la pièce découpée pour masque (110) présentant sur le côté extérieur et sur son bord supérieur (11a) une pièce découpée en feuille (30) comme surface de visibilité en une matière synthétique claire comme du verre, flexible et peu réfléchissante qui est reliée à la pièce découpée pour masque (110) par un assemblage collé ou cousu.

3. Masque pour visage selon la revendication 1, **caractérisé en ce** que le bord d'étanchéité de la pièce découpée pour masque (110) du masque pour visage (10) est constituée par une bande de mousse (120), placée du côté visage sur la pièce découpée pour masque en étant pliée dans le sens transversal par rapport au sens de la longueur, avec une surface collante (21), tournée vers le visage, constituée par une enduction avec une substance autocollante et que le masque pour visage (10) est relié de manière amovible ou fixe à un casque (40), le casque (40) présentant sur le côté extérieur une pièce découpée de type visière (50), fixée de manière fixe sur son bord supérieur (41), comme protection des yeux et contre la réflexion, la pièce découpée (50) étant constituée par une feuille en matière synthétique tentée en noir ou en un autre coloris foncé, opaque ou matée et fixée sur le bord supérieur (41) du casque (40) par des assemblages collés ou cousus.

4. Masque pour visage selon la revendication 1, **caractérisé en ce** que le bord d'étanchéité de la pièce découpée pour masque (110) du masque pour visage (10) est constituée par une bande de mousse (120), placée du côté visage sur la pièce découpée pour masque en étant pliée dans le sens transversal par rapport au sens de la longueur, avec une surface collante (21), tournée vers le visage, constituée par une enduction avec une substance autocollante et que le masque pour visage (10) est relié de manière amovible ou fixe à un casque (40), le casque (40) présentant sur le côté extérieur une pièce découpée de type visière (50), fixée de manière fixe sur son bord supérieur (41), comme protection des yeux et contre la réflexion, la pièce découpée (50) étant constituée par une feuille en matière synthétique teintée en noir ou en un autre coloris foncé, opaque ou matée et fixée sur le bord supérieur (41) du casque (40) au moyen d'une fermeture auto-agrippante.

5. Masque selon l'une des revendications 2 à 4, **caractérisé en ce** que la pièce découpée du type visière (50) présente des bandes de fixation sur ses zones d'extrémités latérales.

Fig.1

Fig.3    Fig.4

EP 0 355 444 B1

# Fig. 2

# Fig. 5

FIG.6